# EUROPEAN PATENT APPLICATION

(11) **EP 4 083 609 A1**
(43) Date of publication of application: **02.11.2022**
(21) Application number: 20904535.0
(22) Date of filing: 04.12.2020
(51) Int. Cl.: G01N 21/3577

(54) **UREA CONCENTRATION SENSOR AND AMMONIA CONCENTRATION SENSOR**

(30) Priority: 24.12.2019 JP 2019233326
(71) Applicant: Kubota Corporation, Osaka 556-8601 (JP)
(72) Inventor: MORIMOTO, Susumu, Amagasaki-shi, Hyogo 661-8567 (JP); MAENO, Daichi, Amagasaki-shi, Hyogo 661-8567 (JP)
(74) Representative: Graf von Stosch Patentanwaltsgesellschaft mbH
(86) International application number: PCT/JP2020/045263
(87) International publication number: WO 2021/131616

(57) **Abstract**

Provided is a urea solution sensor that can accurately measure a concentration of urea. The ammonia concentration sensor (1) includes: a light source (10) that emits measurement light toward a measurement subject, the measurement light including near-infrared light; a light reception unit (20) that receives transmitted light or reflected light from the measurement subject; and an analysis unit (30) that analyzes a concentration of urea contained in the measurement subject based on a spectrum of light which has been received by the light reception unit (20).

## Description

### Technical Field

The present invention relates to a urea concentration sensor and an ammonia concentration sensor.

### Background Art

Patent Literature 1 discloses a diesel engine equipped with a selective reduction type catalyst that has a property of selectively causing NOx (nitrogen oxide) to react with a reducing agent even in the presence of oxygen and that is provided in the middle of an exhaust pipe through which an exhaust gas passes. It is also indicated that a urea solution which acts as a required amount of a reducing agent is added upstream of this selective reduction type catalyst to cause the urea solution to undergo a reduction reaction with NOx in the exhaust gas on the selective reduction type catalyst, and thus an emission concentration of NOx can be reduced.

The urea solution is stored in a urea solution tank provided in a vehicle. However, in the urea solution tank, gradual decomposition of urea in the urea solution generates toxic vapors of ammonia and CO. For this reason, concentrations of these vapors increase in the tank as an operation period of the vehicle increases. In view of this, in the invention disclosed in Patent Literature 1, a concentration of the urea solution is measured, and a vapor generated in the tank is discharged to the outside of the tank as needed.

As a technique for measuring a concentration of a urea solution, Patent Literature 2 discloses a method of thermal conductivity type for measuring a concentration of a urea solution in which an output value corresponding to thermal conductivity and an output value corresponding to kinematic viscosity are measured for a measurement subject liquid, and the concentration of the urea solution is measured from calibration curve data indicating a relation between the output value corresponding to thermal conductivity and the output value corresponding to kinematic viscosity.

### Citation List

### [Patent Literature]

[Patent Literature 1]
   Japanese Patent Application Publication Tokukai No. 2005-076575
[Patent Literature 2]
   Japanese Patent Application Publication Tokukai No. 2008-209405

### Summary of Invention

### Technical Problem

However, in the measurement method of thermal conductivity type described in Patent Literature 2, there is a problem that heat diffusion occurs due to movement of the urea solution by vibrations, and an output value corresponding to thermal conductivity cannot be measured accurately. Therefore, according to the above described measurement method of thermal conductivity type, it is difficult to appropriately measure a concentration of a urea solution from calibration curve data in a urea solution tank or the like which is provided in a vehicle and vibrates.

An aspect of the present invention is accomplished in view of the above problems, and its object is to provide a urea concentration sensor and the like capable of accurately measuring a concentration of a urea solution.

### Solution to Problem

In order to attain the object, a urea concentration sensor in accordance with an aspect of the present invention includes: a light source that emits measurement light toward a measurement subject, the measurement light including near-infrared light; a light reception unit that receives transmitted light or reflected light from the measurement subject; and an analysis unit that analyzes a concentration of urea contained in the measurement subject based on a spectrum of light which has been received by the light reception unit.

According to the configuration, the urea concentration sensor irradiates the measurement subject with the measurement light, and the analysis unit analyzes the concentration of urea based on transmitted light or reflected light received by the light reception unit. Therefore, the urea concentration sensor can reduce influence of thermal diffusion caused due to movement of the measurement subject by vibrations, and it is therefore possible to accurately measure the concentration of urea contained in the measurement subject.

In the urea concentration sensor in accordance with an aspect of the present invention, it is preferable that a wavelength of the measurement light is less than 1100 nm.

According to the configuration, the urea concentration sensor irradiates the measurement subject with the measurement light having a wavelength of less than 1100 nm, and the analysis unit analyzes the concentration of urea based on transmitted light or reflected light received by the light reception unit. Light having a wavelength of less than 1100 nm is less absorbed by water than light having a wavelength of 1100 nm or more. Therefore, by employing light having a wavelength of less than 1100 nm as the measurement light, it is possible to measure a wider range of a measurement subject when the measurement subject contains water.

In the urea concentration sensor in accordance with an aspect of the present invention, it is preferable that the light reception unit receives the transmitted light which has passed through the measurement subject only by a predetermined distance.

According to the configuration, the absorbance and the concentration of urea contained in a measurement subject become proportional to each other, and it is therefore possible to stably analyze a concentration of a urea solution.

In the urea concentration sensor in accordance with an aspect of the present invention, it is preferable that the predetermined distance is 5 mm or more and 50 mm or less.

According to the configuration, it is possible to more stably analyze a concentration of urea.

The urea concentration sensor in accordance with an aspect of the present invention preferably further includes a transmission path for transmitting light between the light source and the light reception unit.

According to the configuration, in the urea concentration sensor in accordance with an aspect of the present invention, a light path between the light source and the light reception unit can be arbitrarily designed.

In the urea concentration sensor in accordance with an aspect of the present invention, it is preferable that: the transmission path includes a first optical fiber for guiding light, which has been emitted by the light source, to the measurement subject; and the transmission path includes a second optical fiber for guiding light, which has passed through the measurement subject, to the light reception unit.

According to the configuration, by bending the first optical fiber or the second optical fiber so as to be arranged in an intended manner, it is possible to appropriately measure a measurement subject in either of a transmission method or an interactance method.

The urea concentration sensor in accordance with an aspect of the present invention preferably further includes: a container that stores the measurement subject; and a lid, only the transmission path being fixed inside the container.

According to the configuration, only the transmission path is immersed in the measurement subject, and the light source, the light reception unit, and the analysis unit are not immersed in the measurement subject. It is therefore possible to reduce a possibility that the urea concentration sensor is deteriorated by urea.

In the urea concentration sensor in accordance with an aspect of the present invention, it is preferable that at least one of the light source, the light reception unit, and the analysis unit is fixed to the lid.

According to the configuration, in the urea concentration sensor in accordance with an aspect of the present invention, at least one of the light source, the light reception unit, and the analysis unit can be fixed at a position higher than the measurement subject without using a separate member. Therefore, even in a case where the measurement subject has leaked out from the container, it is possible to reduce a possibility that the urea concentration sensor is deteriorated by the leaked measurement subject.

The urea concentration sensor in accordance with an aspect of the present invention preferably further includes a temperature sensor that measures a temperature of the measurement subject, the analysis unit analyzing the concentration of urea based on the spectrum which has been corrected based on the temperature measured by the temperature sensor.

According to the configuration, even in a case where a temperature of a measurement subject is changed, it is possible to appropriately analyze a concentration of urea contained in the measurement subject.

In the urea concentration sensor in accordance with an aspect of the present invention, it is preferable that the analysis unit further analyzes, based on the spectrum, a concentration of ammonia contained in the measurement subject.

According to the configuration, it is possible to take into consideration a concentration of ammonia generated by hydrolysis of a urea solution, and it is therefore possible to more appropriately analyze a concentration of the urea solution.

An ammonia concentration sensor in accordance with an aspect of the present invention includes: a light source that emits measurement light toward a measurement subject, the measurement light including near-infrared light; a light reception unit that receives transmitted light or reflected light from the measurement subject; and an analysis unit that analyzes a concentration of ammonia contained in the measurement subject based on a spectrum of light which has been received by the light reception unit.

According to the configuration, it is possible to accurately measure a concentration of ammonia contained in a measurement subject, with a principle similar to that of the foregoing urea concentration sensor.

### Advantageous Effects of Invention

According to an aspect of the present invention, it is possible to provide a urea concentration sensor and the like capable of accurately measuring a concentration of a urea solution.

### Brief Description of Drawings

Fig. 1 is a diagram schematically illustrating an exhaust gas purification system including a urea concentration sensor in accordance with Embodiment 1.
Fig. 2 is a diagram schematically illustrating the urea concentration sensor in accordance with Embodiment 1.
Fig. 3 is a block diagram illustrating a configuration of main parts of the urea concentration sensor in accordance with Embodiment 1.
Fig. 4 is a graph showing an example of absorption spectra of a urea solution and an ammonia solution.
Fig. 5 is a graphical diagram illustrating a calibration curve indicating a relation between absorbance and a concentration of ammonia in a urea solution.
Fig. 6 is a graphical diagram illustrating a calibration curve indicating a relation between absorbance and a concentration of urea in a urea solution.
Fig. 7 is a block diagram illustrating a configuration of main parts of a urea concentration sensor in accordance with Embodiment 2.
Fig. 8 is a diagram schematically illustrating an exhaust gas purification system including a urea concentration sensor in accordance with Embodiment 4.
Fig. 9 is a diagram illustrating a configuration of the urea concentration sensor in accordance with Embodiment 4.
Fig. 10 is a diagram illustrating a configuration of the urea concentration sensor in accordance with Embodiment 4.

### Description of Embodiments

### Embodiment 1

The following description will discuss details of an embodiment of the present invention.

### (Configuration of exhaust gas purification system 100)

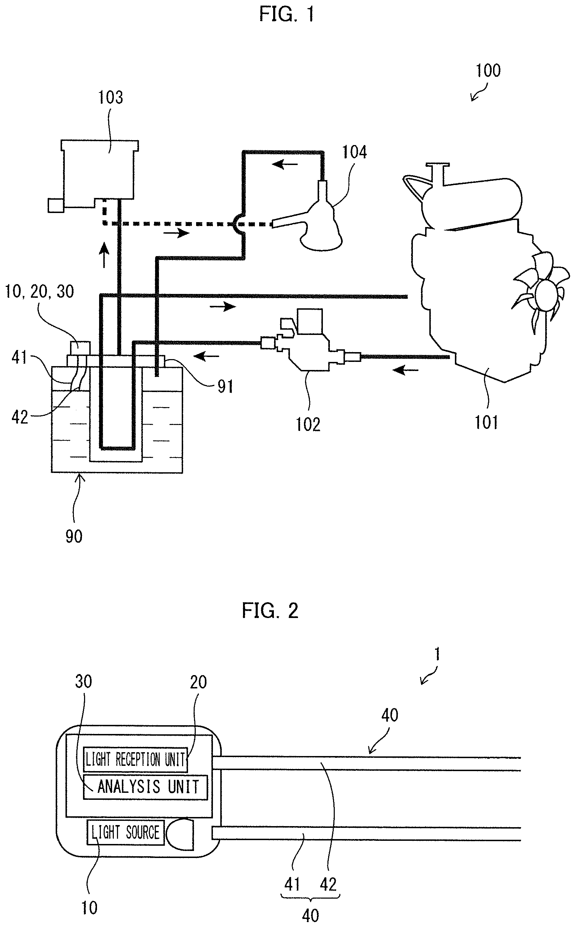

Fig. 1 is a diagram schematically illustrating an exhaust gas purification system 100 including a urea concentration sensor 1 in accordance with Embodiment 1. As illustrated in Fig. 1, the exhaust gas purification system 100 includes an engine 101, a cooling valve 102, a urea solution tank 90 (container) and a lid 91 included in the urea concentration sensor 1, a urea solution pump 103, and a urea solution injector 104.

The engine 101 is a prime mover that burns fuel such as gasoline in a mechanism such as a cylinder, and thus obtains motive power by using a combustion gas generated by the combustion. The combustion gas is discharged as an exhaust gas after the engine 101 has obtained the motive power. The engine 101 is, for example, a diesel engine. Therefore, the exhaust gas includes NOx and the like. The exhaust gas purification system 100 reduces the exhaust gas using a urea solution as a reducing agent. The cooling valve 102 is a device for controlling a flow direction, pressure, or a flow rate of fluid. The cooling valve 102 cools the exhaust gas which has been burnt by the engine 101.

The urea solution tank 90 is a container for storing a urea solution used as a reducing agent. The urea solution tank 90 has a cylindrical shape with one end which is an opening. Note, however, that the urea solution tank 90 is not limited to this shape, provided that the urea solution tank 90 is capable of storing a urea solution. The lid 91 closes the one end of the urea solution tank 90. This makes it possible to reduce a possibility that the urea solution stored in the urea solution tank 90 leaks to the outside of the urea solution tank 90.

The urea solution pump 103 is a device for pumping up the urea solution stored inside the urea solution tank 90 and sending the urea solution to the urea solution injector 104. The urea solution injector 104 is a device for injecting the urea solution, which has been pumped up by the urea solution pump 103 from the urea solution tank 90, toward an exhaust gas discharged from the engine 101.

### (Flow of urea solution)

The urea solution stored in the urea solution tank 90 is pumped up by the urea solution pump 103 and is sent to the urea solution injector 104. Further, the urea solution is injected from the urea solution injector 104 toward an exhaust gas which has been cooled by the cooling valve 102. A part of the urea solution becomes ammonia by hydrolysis and reduces NOx. As a result, a concentration of NOx in the exhaust gas can be lowered. A part of the urea solution injected by the urea solution injector 104 is stored in the urea solution tank 90 again. Thus, the urea solution can be effectively utilized.

### (Configuration of urea concentration sensor 1)

Fig. 2 is a diagram schematically illustrating the urea concentration sensor 1. The urea concentration sensor 1 is a sensor for measuring a concentration of urea in the urea solution stored in the urea solution tank 90. As illustrated in Fig. 2, the urea concentration sensor 1 includes a light source 10, a light reception unit 20, an analysis unit 30, and a transmission path 40.

The light source 10 emits measurement light including near-infrared light toward a measurement subject. The measurement subject is the urea solution stored in the urea solution tank 90. Note, however, that the measurement subject can further include ammonia, and can be a reducing agent containing urea.

In Embodiment 1, measurement light is light having a single or broadband wavelength, such as laser light or light from a light emitting diode (LED). This makes it possible to reduce influence of thermal diffusion caused due to movement of the measurement subject by vibrations, and it is therefore possible to carry out measurement even in a case where the urea solution is flowing. The wavelength of the measurement light is less than 1100 nm. Light having a wavelength of less than 1100 nm is less absorbed by water than light having a wavelength of 1100 nm or more, and therefore a predetermined distance (described later) that transmitted light passes through the measurement subject can be increased. By increasing the distance that transmitted light passes through and measuring the urea solution in a wider range, influence of impurities and the like can be reduced, and a concentration of urea can be accurately measured.

The wavelength of the measurement light is more preferably less than 1000 nm. This makes it possible to more accurately measure a concentration of urea contained in the measurement subject, as compared with a case where the wavelength of the measurement light is 1000 nm or more and less than 1100 nm. Note, however, that, if the wavelength of the measurement light is shorter than 500 nm, the measurement light is refracted due to influence of water, which can affect the measurement of the concentration of urea. Therefore, the wavelength of the measurement light is preferably greater than 500 nm, and more preferably greater than 700 nm. By using measurement light having such wavelengths, influence of refraction by water can be reduced.

The light reception unit 20 receives the transmitted light which has passed through the measurement subject only by a predetermined distance. Intensity of measurement light is known, and therefore absorbance can be calculated from intensity of light received by the light reception unit and based on the Lambert-Beer's law. The absorbance is proportional to the concentration of urea or ammonia contained in a urea solution, and therefore the concentration of urea or ammonia can be analyzed based on the absorbance.

The predetermined distance is preferably 5 mm or more and 50 mm or less, more preferably 10 mm or more and 30 mm or less. By setting the predetermined distance to the above distance, it is possible to analyze the concentration of urea or ammonia more stably. Note, however, that the light reception unit 20 is not limited to a unit that receives transmitted light, and can be configured to receive reflected light reflected by the measurement subject or reflected light via a reflector. That is, the light reception unit 20 only needs to be configured to receive transmitted light or reflected light from a urea solution.

The analysis unit 30 analyzes the concentration of urea and ammonia contained in the urea solution based on a spectrum of light received by the light reception unit 20. A specific configuration of the analysis unit 30 will be described later.

The transmission path 40 is a light guide member for transmitting light between the light source 10 and the light reception unit 20. Thus, in the urea concentration sensor 1, a light path between the light source 10 and the light reception unit 20 can be arbitrarily designed.

In Embodiment 1, the transmission path 40 includes (i) a first optical fiber 41 for guiding light, which has been emitted by the light source 10, to the measurement subject, and (ii) a second optical fiber 42 for guiding light, which has passed through the measurement subject, to the light reception unit 20. One end of the first optical fiber 41 (first entrance end) is provided at a position where light from the light source 10 enters. One end of the second optical fiber 42 (second emission end) is provided at a position where light is emitted toward the light reception unit 20. The other end of the first optical fiber 41 (first emission end) and the other end of the second optical fiber 42 (second entrance end) are fixed in the urea solution tank 90 so as to face each other while keeping the predetermined distance therebetween in a urea solution. With the configuration, transmitted light, which is measurement light emitted from the first emission end and passed through the urea solution by the predetermined distance, enters the second entrance end.

The transmission path 40 is not limited to this, as long as light is transmitted between the light source 10 and the light reception unit 20. For example, the transmission path 40 can be provided with an optical member for guiding light, which has been emitted by the light source 10, while reflecting the light in the optical member.

In the above example, the light reception unit 20 receives the transmitted light which has passed through the urea solution only by the predetermined distance. In other words, the urea concentration sensor 1 measures the urea concentration by a so-called transmission method in which a urea solution is irradiated with measurement light from one side of the urea solution, and light passed through the urea solution by the predetermined distance is detected on the other side. Note, however, that the urea concentration sensor 1 can carry out measurement by an interactance method instead of the transmission method, depending on arrangements of the first optical fiber 41 and the second optical fiber 42. The interactance method refers to a method of detecting measurement light which has been diffused or reflected inside a measurement subject.

### (Attachment position of urea concentration sensor 1)

In the example illustrated in Fig. 1, only the first optical fiber 41 and the second optical fiber 42 among the light source 10, the light reception unit 20, the analysis unit 30, the first optical fiber 41, and the second optical fiber 42 are fixed inside the urea solution tank 90. The light source 10, the light reception unit 20, and the analysis unit 30 are provided outside the urea solution tank 90. For example, the light source 10, the light reception unit 20, and the analysis unit 30 can be provided in a device other than the urea solution tank 90, such as a vehicle body or a fuel tank. This configuration reduces a possibility that the light source 10, the light reception unit 20, and the analysis unit 30 are deteriorated by urea contained in a urea solution stored in the urea solution tank 90 or by ammonia generated by hydrolysis of the urea.

In the example illustrated in Fig. 1, the light source 10, the light reception unit 20, and the analysis unit 30 included in the urea concentration sensor 1 are fixed to the lid 91. According to the configuration, in the urea concentration sensor 1, the light source 10, the light reception unit 20, and the analysis unit 30 can be fixed at a position higher than a liquid level of a urea solution in the urea solution tank 90 without using a separate member. Therefore, even if a urea solution leaks from the urea solution tank 90, it is possible to reduce a possibility that the urea solution adheres to and deteriorates the light source 10, the light reception unit 20, and the analysis unit 30.

Note, however, that, in the urea concentration sensor 1, it is not necessary that all of the light source 10, the light reception unit 20, and the analysis unit 30 are fixed to the lid 91. As long as at least one of the light source 10, the light reception unit 20, and the analysis unit 30 is fixed to the lid 91, it is possible to reduce a possibility of deterioration of that member due to adhesion of the urea solution thereto.

Note that the urea concentration sensor 1 does not necessarily need to be attached to the lid 91, and can be attached to an outer wall of the urea solution tank 90, or to a flow channel for a urea solution which is pumped up by the urea solution pump 103. In such cases, the urea concentration sensor 1 does not necessarily need to include the transmission path 40. In a case where the transmission path 40 is not provided, the urea concentration sensor 1 can measure the concentration of urea with the foregoing interactance method by providing a light source and a light reception unit on, for example, a wall surface of the urea solution tank 90 or of the flow channel for a urea solution.

### (Configuration of analysis unit 30)

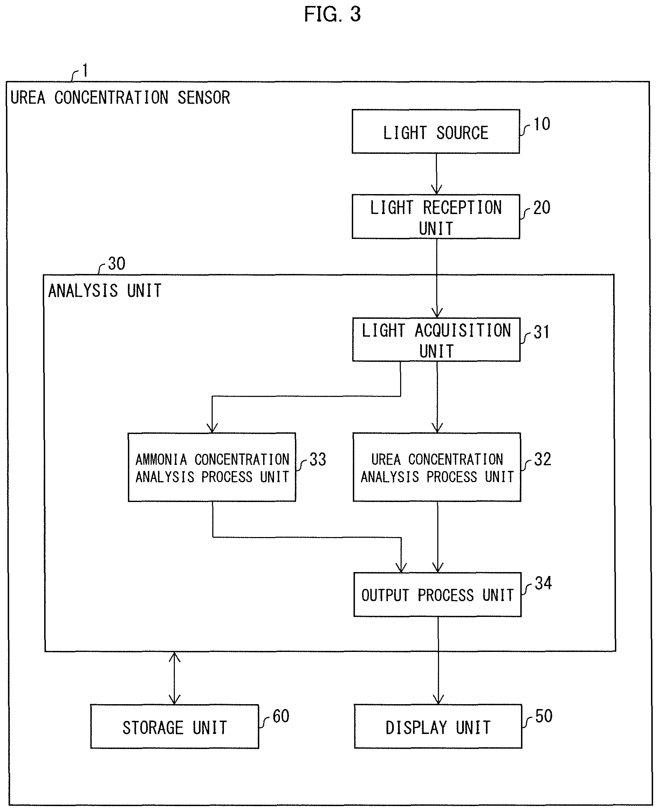

Fig. 3 is a block diagram illustrating a configuration of main parts of the urea concentration sensor 1. As illustrated in Fig. 3, the urea concentration sensor 1 further includes a display unit 50 and a storage unit 60. The analysis unit 30 includes a light acquisition unit 31, a urea concentration analysis process unit 32, an ammonia concentration analysis process unit 33, and an output process unit 34.

The light acquisition unit 31 receives a signal indicating a spectrum of transmitted light received by the light reception unit 20. The light acquisition unit 31 transmits a signal indicating a spectrum of transmitted light to the ammonia concentration analysis process unit 33 and the urea concentration analysis process unit 32. Note that, in a case where a concentration of urea and a concentration of ammonia are simultaneously measured, the number of measurement can be one, because the spectrum of transmitted light received by the light reception unit 20 has information concerning absorbance for wavelengths corresponding to both urea and ammonia. In a case where a concentration of urea and a concentration of ammonia are measured at different time intervals, a spectrum of transmitted light can be measured by changing only wavelengths of the light source suitable for measuring the concentration of urea and the concentration of ammonia, respectively, without adding the first optical fiber 41 and the second optical fiber 42.

The urea concentration analysis process unit 32 analyzes a concentration of urea contained in a urea solution based on a signal indicating a spectrum of transmitted light. Specifically, the urea concentration analysis process unit 32 calculates absorbance at a predetermined wavelength (e.g., 770 nm) from a spectrum of transmitted light. Further, the urea concentration analysis process unit 32 analyzes a concentration of urea contained in a urea solution based on the absorbance and calibration curve data which (i) indicates a relation between absorbance and a concentration of urea and (ii) is stored in the storage unit 60. Note, however, that the urea concentration analysis process unit 32 is not limited to this, as long as the urea concentration analysis process unit 32 is configured to analyze a concentration of urea contained in a urea solution based on a signal indicating a spectrum of transmitted light. The urea concentration analysis process unit 32 transmits a signal indicating the concentration of the analyzed urea to the output process unit 34.

The ammonia concentration analysis process unit 33 analyzes a concentration of ammonia contained in a urea solution based on a signal indicating a spectrum of transmitted light. Specifically, the ammonia concentration analysis process unit 33 calculates absorbance at a predetermined wavelength (e.g., 790 nm) from a spectrum of transmitted light. Further, the ammonia concentration analysis process unit 33 analyzes a concentration of ammonia contained in a urea solution based on the absorbance and calibration curve data which (i) indicates a relation between absorbance and a concentration of ammonia and (ii) is stored in the storage unit 60. Note, however, that the ammonia concentration analysis process unit 33 is not limited to this, as long as the ammonia concentration analysis process unit 33 is configured to analyze a concentration of ammonia contained in a urea solution based on a signal indicating a spectrum of light. The ammonia concentration analysis process unit 33 transmits a signal indicating the concentration of the analyzed ammonia to the output process unit 34.

The output process unit 34 receives, from the urea concentration analysis process unit 32, a signal indicating a concentration of urea. Moreover, the output process unit 34 receives, from the ammonia concentration analysis process unit 33, a signal indicating a concentration of ammonia. The output process unit 34 outputs signals indicating respective concentrations of urea and ammonia to the display unit 50.

The output process unit 34 can also output signals indicating respective concentrations of urea and ammonia to another external device. For example, the output process unit 34 outputs the signals to the urea solution injector 104. The urea solution injector 104 adjusts an injection amount of a urea solution to an exhaust gas based on the signals.

The display unit 50 displays images showing the concentrations of urea and ammonia supplied from the output process unit 34. The display unit 50 is, for example, a liquid crystal display. Note, however, that the urea concentration sensor 1 does not necessarily need to include the display unit 50, and can include a light-emitting element that is turned on or off depending on whether or not the urea concentration is within a predetermined range. Moreover, the urea concentration sensor 1 can include a sound output unit for outputting sound depending on whether or not the urea concentration is within a predetermined range. Alternatively, the urea concentration sensor 1 can be configured to be able to communicate with a display unit, a light-emitting element, or a sound output unit, which is externally provided.

The storage unit 60 is a storage device that stores information necessary for a process by the analysis unit 30. For example, the storage unit 60 stores data relating to absorbance of a urea solution and absorbance of ammonia with respect to wavelengths. The storage unit 60 stores calibration curve data indicating a relation between a concentration and absorbance of urea at a particular wavelength (e.g., 770 nm). The storage unit 60 stores calibration curve data indicating a relation between a concentration and absorbance of ammonia at a particular wavelength (e.g., 790 nm). Note, however, that the urea concentration sensor 1 does not necessarily need to include the storage unit 60, and can be configured to be able to communicate with a storage device which is externally provided.

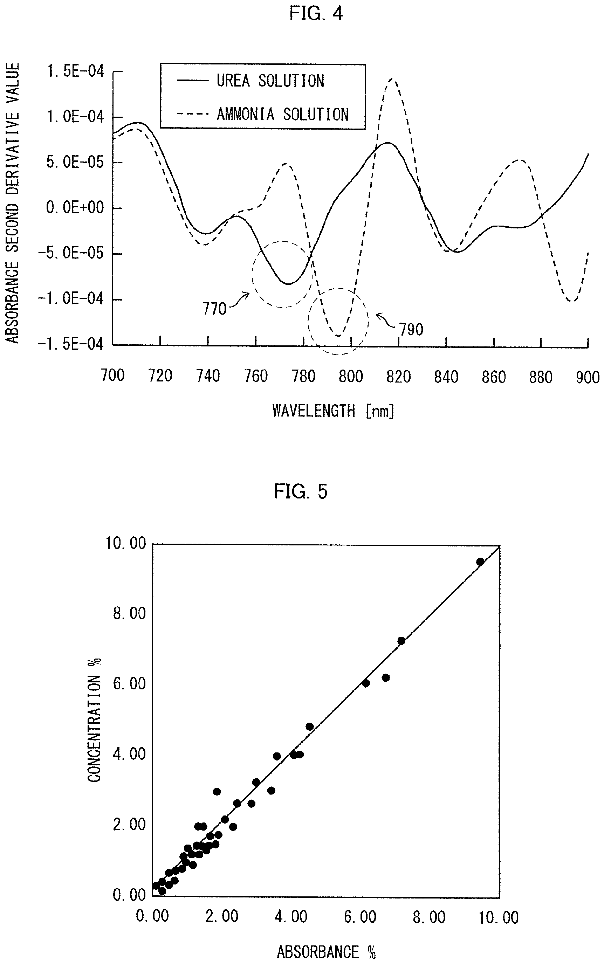

Fig. 4 is a graph showing an example of absorption spectra of a urea solution and an ammonia solution. In the graph illustrated in Fig. 4, the horizontal axis represents wavelengths of transmitted light from a urea solution and an ammonia solution, and the vertical axis represents absorbance second derivative values of the urea solution and the ammonia solution. The absorbance second derivative value is a value corresponding to absorbance and is a value for more accurately measuring absorbance.

In the example illustrated in Fig. 4, an absorption spectrum of a urea solution has a strong negative peak (indicated by the reference sign "770") at a wavelength in the vicinity of 770 nm. Moreover, an absorption spectrum of an ammonia solution has a strong negative peak (indicated by the reference sign "790") at a wavelength in the vicinity of 790 nm. In general, it is preferable to measure absorbance in the vicinity of a wavelength at which absorbance varies less with respect to a variation in wavelength, that is, in the vicinity of a peak of absorbance. Therefore, the urea concentration analysis process unit 32 analyzes the concentration of urea using (i) a peak of absorbance at a position with a wavelength of 770 nm and (ii) calibration curve data (described later) for a urea solution. Moreover, the ammonia concentration analysis process unit 33 analyzes the concentration of ammonia using (i) a peak of absorbance at a position with a wavelength of 790 nm and (ii) calibration curve data (described later).

Fig. 5 is a graph illustrating a calibration curve indicating a relation between absorbance and a concentration of ammonia in a urea solution. In the calibration curve data illustrated in Fig. 5, the horizontal axis represents absorbance of ammonia, and the vertical axis represents a concentration of ammonia. The calibration curve data illustrated in Fig. 5 is data for light with a wavelength in the vicinity of 790 nm. As illustrated in Fig. 5, the concentration of ammonia is in proportion to the absorbance of ammonia. The ammonia concentration analysis process unit 33 analyzes the concentration of ammonia contained in a urea solution based on (i) absorbance for light with a wavelength in the vicinity of 790 nm, which has been calculated based on a signal indicating an absorption spectrum of ammonia solution, and (ii) calibration curve data illustrated in Fig. 5.

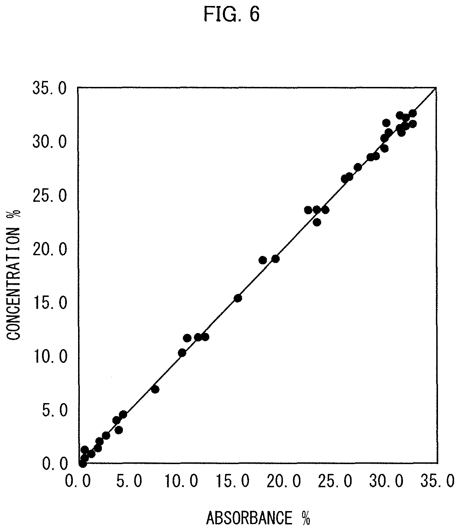

Fig. 6 is a graph illustrating a calibration curve indicating a relation between absorbance and a concentration of urea in a urea solution. In the calibration curve data illustrated in Fig. 6, the horizontal axis represents absorbance of urea, and the vertical axis represents a concentration of urea. The calibration curve data illustrated in Fig. 6 is data for light with a wavelength in the vicinity of 770 nm. As illustrated in Fig. 6, the concentration of urea is in proportion to the absorbance of urea. The urea concentration analysis process unit 32 can analyze the concentration of urea contained in a urea solution based on (i) absorbance for light with a wavelength of 770 nm, which has been calculated based on an absorption spectrum of urea solution, and (ii) calibration curve data illustrated in Fig. 6.

Note that respective absorption spectra of a urea solution and an ammonia solution have peaks corresponding to respective concentrations of urea and ammonia, in addition to the peaks indicated by the reference signs "770" and "790" in Fig. 4. The urea concentration analysis process unit 32 and the ammonia concentration analysis process unit 33 can determine which of the peaks is used to analyze the concentrations of urea and ammonia by calculating absorbance at a plurality of wavelengths at which peaks appear and carrying out multivariate analysis on those calculated values of absorbance. In this case, it is only necessary that the storage unit 60 stores calibration curve data indicating a relation between (i) absorbance at wavelengths which can be used for analyzing concentrations of urea and ammonia and (ii) concentrations of urea and ammonia.

The analysis unit 30 does not necessarily need to include the ammonia concentration analysis process unit 33. The analysis unit 30 can detect a concentration of urea contained in a urea solution, as long as the analysis unit 30 is provided with at least the urea concentration analysis process unit 32.

Note, however, that the analysis unit 30 preferably includes the ammonia concentration analysis process unit 33. That is, the analysis unit 30 preferably analyzes a concentration of ammonia contained in a urea solution based on a spectrum of transmitted light. A part of urea contained in a urea solution can be changed to ammonia while being stored in the urea solution tank 90. In this case, a concentration of urea obtained as a result of analysis by the urea concentration analysis process unit 32 apparently becomes smaller. Therefore, when the urea solution injector 104 has a function of determining an injection amount of a urea solution based on a concentration of urea, there is a possibility that an excessive amount of a urea solution may be injected based on the result of analysis.

When the analysis unit 30 is provided with the ammonia concentration analysis process unit 33, the urea solution injector 104 can determine an amount of a urea solution to be injected, while taking into account an amount of ammonia contained in the urea solution. This reduces the possibility of injecting an excessive amount of a urea solution. In other words, it is possible to appropriately control an amount of a urea solution added to NOx in the exhaust gas purification system 100.

### Embodiment 2

The following description will discuss another embodiment of the present invention. Note that, for convenience, members which have functions identical to those of the members described in Embodiment 1 are given respective identical reference numerals, and such members will not be described again.

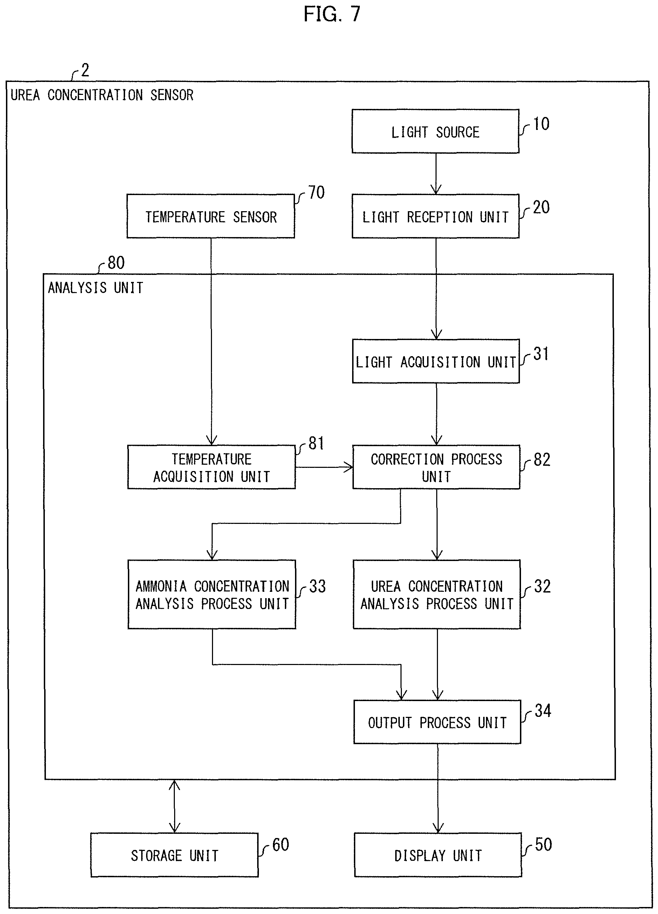

Fig. 7 is a block diagram illustrating a configuration of main parts of a urea concentration sensor 2 in accordance with Embodiment 2. As illustrated in Fig. 7, the urea concentration sensor 2 differs from the urea concentration sensor 1 in that the urea concentration sensor 2 further includes a temperature sensor 70 and includes, instead of the analysis unit 30, an analysis unit 80.

The temperature sensor 70 is a sensor for measuring a temperature of a urea solution. Further, the temperature sensor 70 can measure a temperature of the outside air. In addition to the configuration of the analysis unit 30, the analysis unit 80 further includes a temperature acquisition unit 81 and a correction process unit 82. In the analysis unit 80, the light acquisition unit 31 transmits, to the correction process unit 82, a signal indicating a spectrum of light received by the light reception unit 20. The temperature acquisition unit 81 acquires a signal indicating a temperature of a urea solution from the temperature sensor 70, and outputs the signal to the correction process unit 82.

The correction process unit 82 corrects a signal indicating a spectrum of light based on a signal indicating a spectrum of light, a signal indicating a temperature of a urea solution, and correction data, which is data for correcting the spectrum based on the temperature. The correction data is stored in advance in the storage unit 60. The correction process unit 82 transmits a signal indicating a corrected spectrum of transmitted light to the urea concentration analysis process unit 32 and the ammonia concentration analysis process unit 33. In the analysis unit 80, the urea concentration analysis process unit 32 and the ammonia concentration analysis process unit 33 analyze respective concentrations of urea and ammonia contained in a urea solution on the basis of a signal indicating a spectrum of transmitted light corrected by the correction process unit 82.

Note, however, that the urea concentration sensor 2 does not need to include the temperature sensor 70. In this case, the correction process unit 82 can correct a signal indicating a spectrum of light based on, for example, a signal indicating a spectrum of light and a signal indicating a spectrum of light based on correction data, which is data for correcting the spectrum based on a temperature.

Depending on a temperature of a urea solution, a peak position can shift in a spectrum of transmitted light from the urea solution. The analysis unit 80 includes the correction process unit 82 to analyze a concentration of urea contained in a urea solution based on a spectrum of transmitted light corrected based on the temperature measured by the temperature sensor 70. Thus, even in a case where a temperature of a urea solution is changed, it is possible to appropriately analyze a concentration of urea contained in the urea solution.

The correction process unit 82 can correct a spectrum using a temperature of the outside air. Furthermore, the correction process unit 82 can set a load factor (weight factor) in accordance with a temperature difference between the outside air and a urea solution, and can correct the spectrum based on weighted actual data obtained by multiplying past actual data by the load factor (weight factor). In this case, the load factor in accordance with the temperature difference between the outside air and a urea solution, and the past actual data are stored in advance in the storage unit 60. The storage unit 60 and the machine learning device can be configured to be able to communicate with each other.

The correction process unit 82 can use data processed by the machine learning device (i.e., data indicating which actual data should be multiplied by the load factor) to determine correction data. The machine learning device is configured to receive, for example, product-sum operation values of (i) concentrations and temperatures of a urea solution over a predetermined period in the past and (ii) a load factor in accordance with a temperature difference between the outside air and the urea solution, and to output correction data of a spectrum.

Algorithms used in the machine learning device can include a one-class support vector machine, a local outlier factor (LOF) method, an isolation forest (IF) method, and a robust covariance (RC) method, which are used to predict anomalies caused due to specific combinations.

### Embodiment 3

In each the embodiments described above, the urea concentration sensors 1 and 2 measure at least a concentration of urea. Note, however, that the urea concentration sensors 1 and 2 do not necessarily need to measure a concentration of urea, and can measure, for example, only a concentration of ammonia. In other words, the urea concentration sensors 1 and 2 in the respective embodiments described above can also be used as an ammonia concentration sensor for measuring a concentration of ammonia contained in a measurement subject such as a urea solution.

In this case, the ammonia concentration sensor includes: a light source 10 that emits measurement light toward a measurement subject, the measurement light including near-infrared light; a light reception unit 20 that receives transmitted light or reflected light from the measurement subject; and an analysis unit 30 that analyzes a concentration of ammonia contained in the measurement subject based on a spectrum of light which has been received by the light reception unit 20. According to such an ammonia concentration sensor, it is possible to accurately measure a concentration of ammonia contained in a measurement subject.

### Embodiment 4

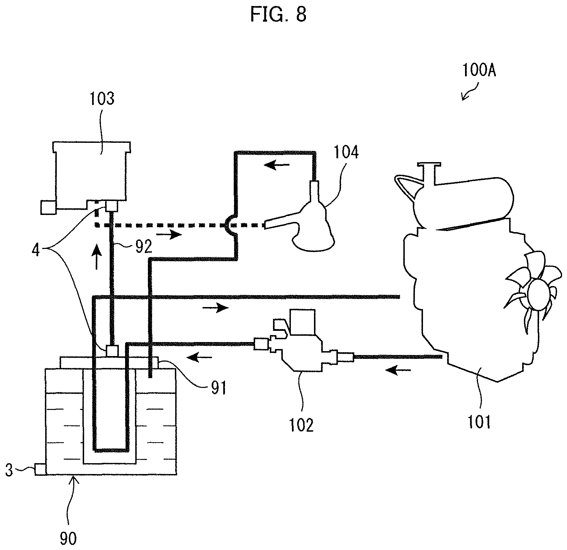

Fig. 8 is a diagram schematically illustrating an exhaust gas purification system 100A including a urea concentration sensors 3 and 4 in accordance with Embodiment 4. The exhaust gas purification system 100A includes one of or both of urea concentration sensors 3 and 4 instead of the urea concentration sensor 1.

As illustrated in Fig. 8, the urea concentration sensor 3 is provided on a side surface on the bottom side of the urea solution tank 90. The urea concentration sensor 4 is provided to a pipe 92. The pipe 92 is a conduit through which a urea solution flows from the urea solution tank 90 to the urea solution pump 103. As illustrated in Fig. 8, the urea concentration sensor 4 can be provided in the vicinity of an end of the pipe 92 on the side of the urea solution tank 90. In other words, the urea concentration sensor 4 can be provided on an upper part of the lid 91. The urea concentration sensor 4 can be provided in the vicinity of an end of the pipe 92 on the side of the urea solution pump 103.

### (Configuration of urea concentration sensor 3)

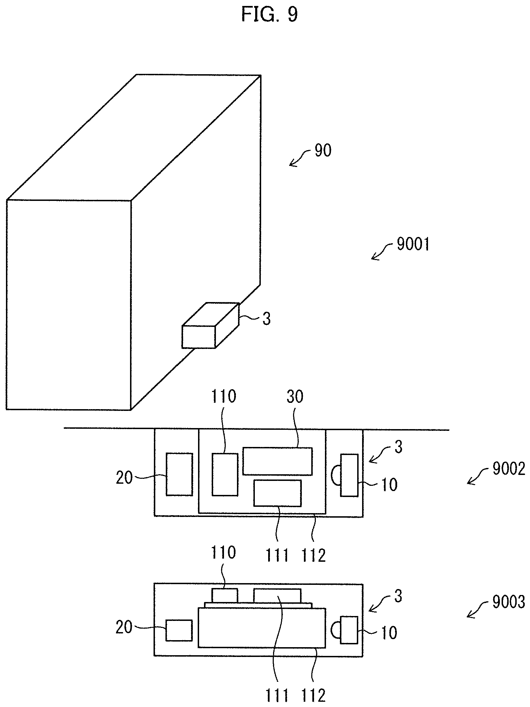

Fig. 9 is a diagram illustrating a configuration of the urea concentration sensor 3. The reference sign "9001" in Fig. 9 is a perspective view illustrating an arrangement of the urea concentration sensor 3. The reference sign "9002" in Fig. 9 is a plan view illustrating the urea concentration sensor 3. The reference sign "9003" in Fig. 9 is a front view illustrating the urea concentration sensor 3. As illustrated in Fig. 9, the urea concentration sensor 3 includes a light source 10, a light reception unit 20, an analysis unit 30, a power source 110, a communication unit 111, and an accommodation unit 112.

The light source 10 is as described above. Specific examples of the light source 10 include, but are not limited to, a broadband LED. The power source 110 is a device for supplying electric power to the urea concentration sensors 3 and 4. The communication unit 111 is a communication device for carrying out communication with an external device. The communication unit 111 has an interface (I/F) for controller area network (CAN) communication. The urea concentration sensor 1 in Embodiment 1 and the like also has a power source and a communication unit, but illustration thereof is omitted.

The accommodation unit 112 is a part in which a urea solution is accommodated so that the analysis unit 30 analyzes a concentration of urea contained in the urea solution. As illustrated in Fig. 9, the accommodation unit 112 is a rectangular parallelepiped container in which an opening is formed on a side facing the urea solution tank 90 so that a urea solution stored in the urea solution tank 90 can flow into the accommodation unit 112. The accommodation unit 112 is constituted by a material that transmits light emitted from the light source 10.

The light source 10 is provided at a position where light enters one side surface of the accommodation unit 112. The light reception unit 20 is provided at a position for receiving light emitted from a side surface of the accommodation unit 112 which is opposite to the side surface at which the light source 10 is provided. Note, however, that the arrangement of the light source 10 and the light reception unit 20 is not limited to this, provided that a function of the light reception unit 20 can be achieved from the outside of the accommodation unit 112.

### (Configuration of urea concentration sensor 4)

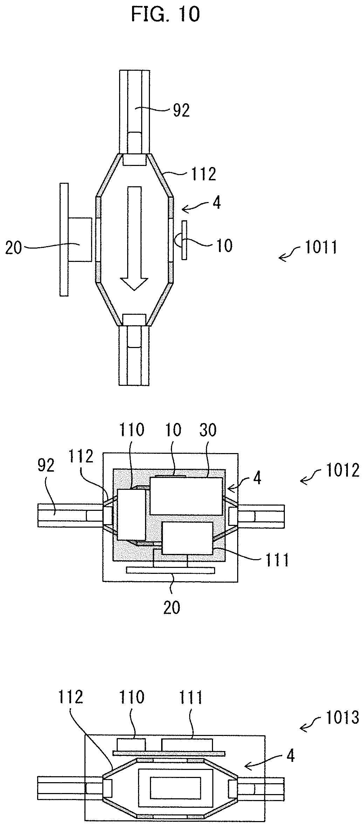

Fig. 10 is a diagram illustrating a configuration of the urea concentration sensor 4. The reference sign "1011" in Fig. 10 is a diagram illustrating an arrangement of the urea concentration sensor 4. The reference sign "1012" in Fig. 10 is a plan view illustrating the urea concentration sensor 4. The reference sign "1013" in Fig. 10 is a front view illustrating the urea concentration sensor 4.

The urea concentration sensor 4 includes a light source 10, a light reception unit 20, an analysis unit 30, a power source 110, a communication unit 111, and an accommodation unit 112, as with the urea concentration sensor 3. As illustrated in Fig. 10, in the urea concentration sensor 4, the accommodation unit 112 is provided in the middle of the pipe 92. Thus, a urea solution flowing through the pipe 92 passes through the accommodation unit 112. As illustrated in Fig. 10, the light source 10 and the light reception unit 20 are arranged to face each other across the accommodation unit 112. Note, however, that the arrangement of the light source 10 and the light reception unit 20 is not limited to this, provided that a function of the light reception unit 20 can be achieved from the outside of the accommodation unit 112.

### (Effects of urea concentration sensors 3 and 4)

In each of the urea concentration sensors 3 and 4, the light source 10 and the light reception unit 20 are provided outside the accommodation unit 112. The analysis unit 30 can analyze concentrations of urea and ammonia contained in the urea solution based on a spectrum of light received by the light reception unit 20. As compared with the urea concentration sensor 1 in accordance with Embodiment 1, each of the urea concentration sensors 3 and 4 can analyze concentrations of urea and ammonia contained in a urea solution by the analysis unit 30 without optical fibers 41 and 42. It is therefore possible to reduce the number of components.

The exhaust gas purification system 100A can include only one of the urea concentration sensors 3 and 4, or can include two or more of the urea concentration sensors 3 and 4. The exhaust gas purification system 100A can include another urea concentration sensor 4 in addition to two urea concentration sensors 4. In this case, said another urea concentration sensor 4 is provided, for example, at a halfway point of the pipe 92 between the urea solution tank 90 and the urea solution pump 103. In a case where the exhaust gas purification system 100A includes two or more of the urea concentration sensors 3 and 4, and a concentration of urea contained in a urea solution analyzed by the analysis unit 30 in the urea concentration sensor 3 is different from that analyzed by the analysis unit 30 in the urea concentration sensor 4, an average of these concentrations can be defined as a concentration of urea contained in the urea solution.

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

1, 2, 3, 4: Urea concentration sensor
10: Light source
20: Light receiving section
30, 80: Analysis unit
40: Transmission path
41: First optical fiber
42: Second optical fiber
70: Temperature sensor
90: Urea solution tank (container)
91: Lid

## Claims

1. A urea concentration sensor, comprising:
a light source that emits measurement light toward a measurement subject, the measurement light including near-infrared light;
a light reception unit that receives transmitted light or reflected light from the measurement subject; and
an analysis unit that analyzes a concentration of urea contained in the measurement subject based on a spectrum of light which has been received by the light reception unit.

2. The urea concentration sensor as set forth in claim 1, wherein a wavelength of the measurement light is less than 1100 nm.

3. The urea concentration sensor as set forth in claim 1 or 2, wherein the light reception unit receives the transmitted light which has passed through the measurement subject only by a predetermined distance.

4. The urea concentration sensor as set forth in claim 3, wherein the predetermined distance is 5 mm or more and 50 mm or less.

5. The urea concentration sensor as set forth in any one of claims 1 through 4, further comprising a transmission path for transmitting light between the light source and the light reception unit.

6. The urea concentration sensor as set forth in claim 5, wherein:
the transmission path includes a first optical fiber for guiding light, which has been emitted by the light source, to the measurement subject; and
the transmission path includes a second optical fiber for guiding light, which has passed through the measurement subject, to the light reception unit.

7. The urea concentration sensor as set forth in claim 5 or 6, further comprising:
a container that stores the measurement subject; and
a lid,
only the transmission path being fixed inside the container.

8. The urea concentration sensor as set forth in claim 7, wherein at least one of the light source, the light reception unit, and the analysis unit is fixed to the lid.

9. The urea concentration sensor as set forth in any one of claims 1 through 8, further comprising a temperature sensor that measures a temperature of the measurement subject,
the analysis unit analyzing the concentration of urea based on the spectrum which has been corrected based on the temperature measured by the temperature sensor.

10. The urea concentration sensor as set forth in any one of claims 1 through 9, wherein the analysis unit further analyzes, based on the spectrum, a concentration of ammonia contained in the measurement subject.

11. An ammonia concentration sensor, comprising:
a light source that emits measurement light toward a measurement subject, the measurement light including near-infrared light;
a light reception unit that receives transmitted light or reflected light from the measurement subject; and
an analysis unit that analyzes a concentration of ammonia contained in the measurement subject based on a spectrum of light which has been received by the light reception unit.
